# EUROPEAN PATENT APPLICATION

(11) **EP 4 678 923 A1**
(43) Date of publication of application: **14.01.2026**
(21) Application number: 23926067.2
(22) Date of filing: 06.12.2023
(51) Int. Cl.: F04D 29/66, A61M 16/00

(54) **NOISE REDUCTION BOX AND VENTILATION THERAPY APPARATUS**

(30) Priority: 07.03.2023 CN 202310214602
(71) Applicant: BMC (Dongguan) Medical Co., Ltd., Dongguan, Guangdong 523000 (CN)
(72) Inventor: CHEN, Yunjing, Dongguan, Guangdong 523000 (CN); WANG, Qingsong, Dongguan, Guangdong 523000 (CN); TIAN, Xin, Dongguan, Guangdong 523000 (CN); ZHUANG, Zhi, Dongguan, Guangdong 523000 (CN)
(74) Representative: Grünecker Patent- und Rechtsanwälte PartG mbB
(86) International application number: PCT/CN2023/136895
(87) International publication number: WO 2024/183373

(57) **Abstract**

A noise reduction box and a ventilation therapy apparatus. The noise reduction box includes a housing (10), a first flow guide pipe (23) and a second flow guide pipe (24). At least two chambers (15) are provided in the housing (10); the first flow guide pipe (23) and the second flow guide pipe (24) are both configured to guide the flow of a gas, so that the gas flows from one of the chambers (15) to another one of the chambers (15). When a gas pressure in a reference chamber is less than or equal to a reference gas pressure threshold, the first flow guide pipe (23) guides the flow of the gas; and when the gas pressure in the reference chamber is greater than the reference gas pressure threshold, the first flow guide pipe (23) and the second flow guide pipe (24) jointly guide the flow of the gas. The noise reduction box can change a gas path according to a change in the rotational speed of a gas pressurization unit, and has a better silencing effect; and the noise reduction box prolongs the service life of the gas pressurization unit while achieving noise reduction.

## Description

The present application claims the priority to Chinese Patent Application No. 202310214602.6, filed with the China National Intellectual Property Administration on March 07, 2023, and entitled "NOISE REDUCTION BOX AND VENTILATION THERAPY APPARATUS", which is incorporated herein by reference in its entirety.

### Technical Field

The present application relates to the field of respiratory apparatuses, and in particular to a noise reduction box and a ventilation therapy apparatus.

### Background Art

Fans are mechanical devices that use input mechanical energy to increase gas pressure and discharge gas. Fans are widely used. For example, both ventilators and high-flow oxygen therapy devices are internally provided with fans.

During operation, fans will generate noise. In the prior art, noise reduction boxes used for installing fans in ventilators and oxygen therapy devices usually employ sound-absorbing cotton for noise reduction, to meet the standard requirements for noise. Due to the properties of the material itself, the sound-absorbing foam may generate fine particles after long-term operation or frequent ozone disinfection. Once the fine particles are inhaled by patients, potential health risks may arise. However, the noise reduction box that does not use sound-absorbing cotton for noise reduction has the disadvantage of a poor noise reduction effect.

### Summary

In view of this, the present application aims to provide a noise reduction box to solve or partially solve the problem of potential health risks and poor noise reduction effect of the existing noise reduction boxes.

To achieve the above-mentioned objective, the technical solutions of the present application are implemented as follows.

A noise reduction box includes a housing, a first flow guide pipe, and a second flow guide pipe. At least two chambers are provided in the housing; the first flow guide pipe and the second flow guide pipe are both configured to guide the flow of a gas, so that the gas flows from one of the chambers to another one of the chambers. When a gas pressure in a reference chamber is less than or equal to a reference gas pressure threshold, the first flow guide pipe guides the flow of the gas; and when the gas pressure in the reference chamber is greater than the reference gas pressure threshold, the first flow guide pipe and the second flow guide pipe jointly guide the flow of the gas.

Further, the noise reduction box further includes an actuating assembly connected to the second flow guide pipe. The actuating assembly is configured to block the second flow guide pipe when the gas pressure in the reference chamber is less than or equal to the reference gas pressure threshold, and to open the second flow guide pipe when the gas pressure in the reference chamber is greater than the reference gas pressure threshold.

Further, an outlet end of the second flow guide pipe is oriented upward; the actuating assembly includes a blocking plate rotatably connected to the outlet end of the second flow guide pipe; and when the gas pressure in the reference chamber is greater than the reference gas pressure threshold, the gas pushes the blocking plate to rotate and open the second flow guide pipe.

Further, the actuating assembly includes a blocking plate and an elastic member; the blocking plate is configured to block an outlet end of the second flow guide pipe; one end of the elastic member is connected to the blocking plate, and the other end of the elastic member is connected to the housing; and when the gas pressure in the reference chamber is greater than the reference gas pressure threshold, the gas pushes the blocking plate to overcome an elastic force of the elastic member and open the second flow guide pipe.

Further, the actuating assembly includes a blocking plate, a pressure monitoring element and an actuating member; the blocking plate is configured to block an inlet end of the second flow guide pipe or an outlet end of the second flow guide pipe; the pressure monitoring element is configured to monitor the gas pressure in the reference chamber and is connected to the actuating member; and when the pressure monitoring element detects that the gas pressure in the reference chamber is greater than the reference gas pressure threshold, the actuating member drives the blocking plate to move and open the second flow guide pipe.

Further, the blocking plate is an elastic material component.

Further, the noise reduction box further includes a partition plate. The partition plate is disposed in the housing, and is connected to the housing, the first flow guide pipe and the second flow guide pipe; the partition plate divides the housing into the at least two chambers; and the first flow guide pipe and the second flow guide pipe both extend out from a surface of the partition plate.

Further, the first flow guide pipe is connected to one of partition plates, and the second flow guide pipe is connected to at least one of the partition plates.

Further, an inner diameter of the first flow guide pipe is smaller than an inner diameter of the second flow guide pipe.

Further, any one of partition plates is provided with at least two first flow guide pipes.

Further, at least two partition plates are arranged in parallel and spaced apart.

Further, the housing is a plastic material component.

Compared with the prior art, the noise reduction box of the present application has the following advantages.

In the noise reduction box of the present application, each chamber in the noise reduction box is equivalent to an expansion chamber. When entering each expansion chamber through the flow guide pipe 26, the gas flow undergoes a silencing process, so as to achieve the purpose of noise reduction. A gas path in the noise reduction box can also change according to the rotational speed of the gas pressurization unit. When the gas pressurization unit is at a low rotational speed, it mainly generates low-frequency noise. At this time, less gas is inhaled by the gas pressurization unit, and the gas pressure in the chambers is relatively low. When the gas pressure in the reference chamber is less than or equal to the reference gas pressure threshold, the first flow guide pipe guides the flow of the gas, and low-frequency noise is reduced by means of the high gas resistance of the first flow guide pipe. When the gas pressurization unit is at a relatively high rotational speed, more gas is inhaled by the gas pressurization unit, and the gas pressure in the chambers increases. When the gas pressure in the reference chamber is greater than the reference gas pressure threshold, the first flow guide pipe and the second flow guide pipe jointly guide the flow of the gas. The ventilation area in the noise reduction box increases, the overall gas resistance decreases, the flow velocity of the gas decreases accordingly, and secondary eddy noise and high-frequency noise caused by the high gas flow velocity also decrease, thereby achieving the purpose of noise reduction. Moreover, the increase of the ventilation area in the noise reduction box reduces the gas resistance in the noise reduction box, and accordingly reduces output power of a gas pressurization unit motor in the gas pressurization unit, reduces a load on the gas pressurization unit, improves the efficiency of the gas pressurization unit, lowers the temperature of the gas pressurization unit, achieves more stable operation of the gas pressurization unit, and prolongs the service life of the gas pressurization unit. That is, the noise reduction box can change the gas path according to a change in the rotational speed of the gas pressurization unit, thereby achieving a better silencing effect; and the noise reduction box prolongs the service life of the gas pressurization unit while achieving noise reduction.

Another objective of the present application is to provide a ventilation therapy apparatus to solve or partially solve the problem of potential health risks and poor noise reduction effect of existing ventilation therapy apparatuses.

To achieve the above-mentioned objective, the technical solutions of the present application are implemented as follows.

A ventilation therapy apparatus includes a gas pressurization unit and the noise reduction box described above, the gas pressurization unit being disposed in the noise reduction box.

Further, the ventilation therapy apparatus further includes a shock-absorbing sleeve. An outer side of the gas pressurization unit is enclosed by the shock-absorbing sleeve, and a suspension end is provided at an upper end of the shock-absorbing sleeve; the gas pressurization unit is suspended from the partition plate via the suspension end, and a portion of the suspension end located between the partition plate and the gas pressurization unit defines an air intake channel; and the air intake channel has one end connected to a partition plate through hole in the partition plate, and the other end connected to a gas pressurization unit gas inlet.

The ventilation therapy apparatus and the noise reduction box described above have the same advantages over the prior art, which will not be repeated here.

The above-mentioned description is merely a summary of the technical solutions of the present application. In order to more clearly understand the technical means of the present application so that the implementation can be carried out according to the content of the description, and in order to make the above-mentioned and other objects, features and advantages of the present application more comprehensible, the particular embodiments of the present application will be illustrated below.

### Brief Description of the Drawings

In order to illustrate the technical solutions of the embodiments of the present application more clearly, the accompanying drawings required for describing the embodiments of the present application will be briefly introduced below. It is clear that the accompanying drawings in the description below show only some embodiments of the present application, and a person of ordinary skill in the art can obtain other accompanying drawings according to these accompanying drawings without creative efforts.
Fig. 1 is a first schematic structural diagram of a noise reduction box according to a first embodiment of the present application;
Fig. 2 is a second schematic structural diagram of the noise reduction box according to the first embodiment of the present application;
Fig. 3 is a first schematic structural diagram of a noise reduction box according to a second embodiment of the present application;
Fig. 4 is a second schematic structural diagram of the noise reduction box according to the second embodiment of the present application;
Fig. 5 is a first schematic structural diagram of a noise reduction box according to a third embodiment of the present application;
Fig. 6 is a second schematic structural diagram of the noise reduction box according to the third embodiment of the present application;
Fig. 7 is a first schematic structural diagram of a noise reduction box according to a fourth embodiment of the present application;
Fig. 8 is a second schematic structural diagram of the noise reduction box according to the fourth embodiment of the present application;
Fig. 9 is a first schematic structural diagram of a noise reduction box according to a fifth embodiment of the present application;
Fig. 10 is a second schematic structural diagram of the noise reduction box according to the fifth embodiment of the present application;
Fig. 11 is a schematic structural diagram of a noise reduction box according to a sixth embodiment of the present application; and
Fig. 12 is a schematic structural diagram of a noise reduction box according to a seventh embodiment of the present application.

List of reference signs:
10 - Housing; 11 - Upper housing; 12 - Intermediate housing; 13 - Lower housing; 14 - Housing gas inlet; 15 - Chamber; 16 - Head-end chamber; 17 - Tail-end chamber; 18 - Intermediate chamber;
20 - Partition plate; 21 - First partition plate; 22 - Second partition plate; 23 - First flow guide pipe; 24 - Second flow guide pipe; 25 - Partition plate through hole; 26 - Flow guide pipe;
31 - First blocking plate; 32 - Second blocking plate; 33 - Elastic member; 34 - Third blocking plate; 36 - Actuating motor; 37 - Fourth blocking plate; 38 - Electromagnet;
40 - Gas pressurization unit; 41 - Gas pressurization unit gas inlet; 42 - Gas pressurization unit gas outlet; 43 - Shock-absorbing sleeve; 44 - Suspension end; 45 - Air intake channel; 46 - Gas pressurization unit motor.

### DETAILED DESCRIPTION

The technical solutions in the embodiments of the present application will be clearly and completely described below in conjunction with the accompanying drawings in the embodiments of the present application. Apparently, the embodiments described are some rather than all of the embodiments of the present application. Based on the embodiments of the present application, all the other embodiments obtained by those of ordinary skill in the art without creative efforts shall fall within the scope of protection of the present application.

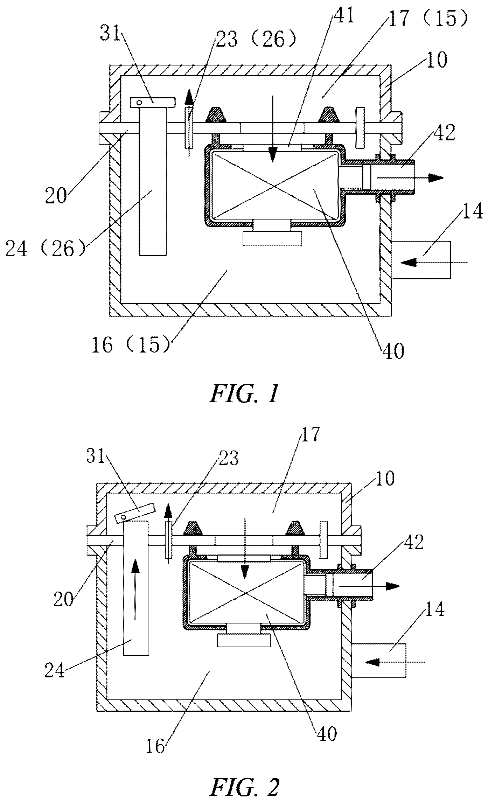

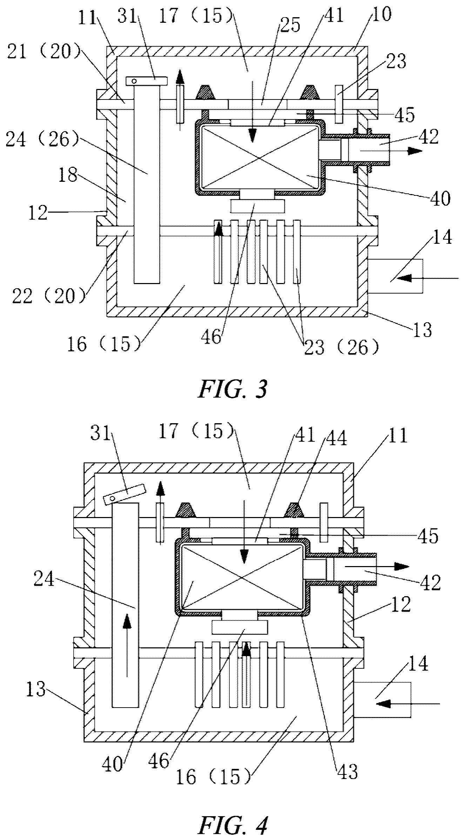

The embodiments of the present application provide a noise reduction box. Referring to Figs. 1 and 3, Fig. 1 is a schematic structural diagram of a noise reduction box according to a first embodiment of the present application, and Fig. 3 is a schematic structural diagram of a noise reduction box according to a second embodiment of the present application. The noise reduction box includes a housing 10 and at least two flow guide pipes 26. The housing 10 is configured to accommodate a gas pressurization unit 40. At least two chambers 15 are provided in the housing 10. The flow guide pipes 26 include a first flow guide pipe 23 and a second flow guide pipe 24. The first flow guide pipe 23 and the second flow guide pipe 24 are both configured to guide the flow of a gas, so that the gas flows from one of the chambers 15 to another one of the chambers 15, allowing the gas within the chambers 15 to flow into the gas pressurization unit 40. When a gas pressure in a reference chamber is less than or equal to a reference gas pressure threshold, the first flow guide pipe 23 guides the flow of the gas; and when the gas pressure in the reference chamber is greater than the reference gas pressure threshold, the first flow guide pipe 23 and the second flow guide pipe 24 jointly guide the flow of the gas.

Specifically, the housing 10 is sealed, and a partition plate 20 is disposed in the housing 10. At least one partition plate 20 divides the interior of the housing 10 into the at least two chambers 15. Referring to Fig. 1, a structure is shown in which one partition plate 20 divides the interior of the housing 10 into two chambers 15. Referring to Fig. 3, a structure is shown in which two partition plates 20 divide the interior of the housing 10 into three chambers 15, and each chamber 15 is a relatively sealed chamber 15.

The housing 10 is provided with a housing gas inlet 14 and a mounting opening. The housing gas inlet 14 is configured to introduce the gas into the housing 10, and the mounting opening is configured to mount a gas pressurization unit gas outlet 42. In a flow direction of the gas, the chamber 15 located at a head end is a head-end chamber 16, and the head-end chamber 16 is in communication with the outside via the housing gas inlet 14 of the housing 10; and the chamber 15 at a tail end is a tail-end chamber 17, and the tail-end chamber 17 is in communication with a gas pressurization unit gas inlet 41 of the gas pressurization unit 40. The gas enters the head-end chamber 16 through the housing gas inlet 14. The gas flows through the at least two chambers 15 and at least one of the flow guide pipes 26 within the housing 10 and eventually flows out of the housing 10 through the gas pressurization unit gas outlet 42 of the gas pressurization unit 40.

Two adjacent chambers 15 are located on two sides of the partition plate 20, and the flow guide pipes 26 are connected to at least one partition plate 20. The flow guide pipes 26 include the first flow guide pipe 23 and the second flow guide pipe 24. The first flow guide pipe 23 is connected to at least one partition plate 20, and the second flow guide pipe 24 is also connected to at least one partition plate 20. For example, referring to Fig. 1, the first flow guide pipe 23 and the second flow guide pipe 24 are both connected to one partition plate 20, and both the first flow guide pipe 23 and the second flow guide pipe 24 are in communication with the chambers 15 on both sides of the partition plate 20. As another example, referring to Fig. 3, the first flow guide pipe 23 is configured to penetrate through the partition plate 20, and the chambers 15 on both sides of the partition plate 20 are in communication with each other via the first flow guide pipe 23. The second flow guide pipe 24 is configured to penetrate through two partition plates 20. One end of the second flow guide pipe 24 is located within the head-end chamber 16, and the other end of the second flow guide pipe 24 is located within the tail-end chamber 17. The head-end chamber 16 and the tail-end chamber 17 are in communication with each other via the second flow guide pipe 24.

The reference chamber is at least one of the at least two chambers 15. The reference chamber is specifically selected according to usage requirements. For example, the reference chamber may be the head-end chamber 16 or the tail-end chamber 17.

In the noise reduction box of the embodiments of the present application, the chambers 15 are equivalent to expansion chambers. After entering each chamber 15 through the first flow guide pipe 23 or the second flow guide pipe 24, the gas flow undergoes a silencing process, so that the purpose of noise reduction can be achieved.

The noise reduction box of the embodiments of the present application uses the chambers 15 and the flow guide pipes 26 to achieve noise reduction without using sound-absorbing cotton, thus providing improved safety and environmental friendliness. When the gas enters the gas pressurization unit 40, noise is generated at the gas pressurization unit gas inlet 41. During the outward propagation, the noise passes through the chambers 15 and the flow guide pipes 26. The chambers 15 and the flow guide pipes 26 have a blocking effect on the noise and convert part of acoustic energy of the noise into thermal energy, weakening the acoustic energy and thus reducing the noise.

In the noise reduction box of the embodiments of the present application, each chamber 15 in the noise reduction box is equivalent to an expansion chamber. When entering each expansion chamber through the flow guide pipe 26, the gas flow undergoes a silencing process, so as to achieve the purpose of noise reduction. A gas path in the noise reduction box can also change according to the rotational speed of the gas pressurization unit 40. When the gas pressurization unit 40 is at a low rotational speed, it mainly generates low-frequency noise. At this time, less gas is inhaled by the gas pressurization unit 40, and the gas pressure in the chambers 15 is relatively low. When the gas pressure in the reference chamber is less than or equal to the reference gas pressure threshold, the first flow guide pipe 23 guides the flow of the gas, and low-frequency noise is reduced by means of the high gas resistance of the first flow guide pipe 23. When the gas pressurization unit 40 is at a relatively high rotational speed, more gas is inhaled by the gas pressurization unit 40, and the gas pressure in the chambers 15 increases. When the gas pressure in the reference chamber is greater than the reference gas pressure threshold, the first flow guide pipe 23 and the second flow guide pipe 24 jointly guide the flow of the gas. The ventilation area in the noise reduction box increases, the overall gas resistance decreases, the flow velocity of the gas decreases accordingly, and secondary eddy noise and high-frequency noise caused by the high gas flow velocity also decrease, thereby achieving the purpose of noise reduction. Moreover, the increase of the ventilation area in the noise reduction box reduces the gas resistance in the noise reduction box, and accordingly reduces output power of a gas pressurization unit motor 46 in the gas pressurization unit 40, reduces a load on the gas pressurization unit 40, improves the efficiency of the gas pressurization unit 40, lowers the temperature of the gas pressurization unit 40, achieves more stable operation of the gas pressurization unit 40, and prolongs the service life of the gas pressurization unit 40. That is, the noise reduction box can change the gas path according to a change in the rotational speed of the gas pressurization unit 40, thereby achieving a better silencing effect; and the noise reduction box prolongs the service life of the gas pressurization unit 40 while achieving noise reduction.

In some embodiments of the present application, the noise reduction box further includes an actuating assembly connected to the second flow guide pipe 24. The actuating assembly is configured to block the second flow guide pipe 24 when the gas pressure in the reference chamber is less than or equal to the reference gas pressure threshold, and to open the second flow guide pipe 24 when the gas pressure in the reference chamber is greater than the reference gas pressure threshold.

Specifically, the gas pressure in the reference chamber affects the operation of the actuating assembly. When the gas pressurization unit 40 is at a relatively high rotational speed, the gas pressure in the reference chamber is greater than the reference gas pressure threshold. After the actuating assembly opens the second flow guide pipe 24 blocked by the actuating assembly, the second flow guide pipe 24 is involved in gas delivery. The ventilation area in the noise reduction box increases, the overall gas resistance decreases, the flow velocity of the gas decreases accordingly, and secondary eddy noise and high-frequency noise caused by the high gas flow velocity also decrease, thereby achieving the purpose of noise reduction.

The noise reduction box of the embodiments of the present application includes two gas paths. The first gas path communicates the first flow guide pipe 23 with the chambers 15, and the second gas path communicates the second flow guide pipe 24 with the chambers 15. When the gas pressure in the reference chamber is less than or equal to the reference gas pressure threshold, the actuating assembly blocks the second flow guide pipe 24, only the first flow guide pipe 23 is open, and the first gas path is an open gas path. When the gas pressure in the reference chamber is greater than the reference gas pressure threshold, the actuating assembly opens the second flow guide pipe 24, the first flow guide pipe 23 and the second flow guide pipe 24 are both open, and both the first gas path and the second gas path are open gas paths.

In some embodiments of the present application, referring to Figs. 1 and 3, an outlet end of the second flow guide pipe 24 is oriented upward. The actuating assembly includes a first blocking plate 31. The first blocking plate 31 is rotatably connected to the outlet end of the second flow guide pipe 24. When the gas pressure in the reference chamber is greater than the reference gas pressure threshold, the gas pushes the first blocking plate 31 to rotate and open the second flow guide pipe 24. The reference chamber is the head-end chamber 16.

In one specific embodiment of the present application, referring to Figs. 1 and 2, Fig. 1 shows a schematic structural diagram of the noise reduction box according to the first embodiment of the present application in which the first blocking plate 31 blocks the outlet end of the second flow guide pipe 24, and Fig. 2 shows a schematic structural diagram of the noise reduction box according to the first embodiment of the present application in which the first blocking plate 31 opens the outlet end of the second flow guide pipe 24. The first blocking plate 31 is connected to one side of the outlet end of the second flow guide pipe 24. For example, the first blocking plate 31 is rotatably connected to a left side of the outlet end as shown in Fig. 1.

Referring to Fig. 1, when the gas pressurization unit 40 is at a low rotational speed, the gas pressure in the head-end chamber 16 is less than or equal to the reference gas pressure threshold. The first blocking plate 31 blocks the second flow guide pipe 24 under the action of its own weight or, for example, under the action of the elastic force of a torsion spring. As shown in Fig. 1, the gas passes through the housing gas inlet 14 into the head-end chamber 16, passes through the first flow guide pipe 23 into the tail-end chamber 17 and then enters the gas pressurization unit gas inlet 41. The gas pressurization unit 40 rotates to accelerate the gas, and the gas flow is then expelled through the gas pressurization unit gas outlet 42. Referring to Fig. 2, when the gas pressurization unit 40 is at a relatively high rotational speed, the gas pressure in the head-end chamber 16 is greater than the reference gas pressure threshold, and the force of the gas acting on the first blocking plate 31 can push the first blocking plate 31 to rotate and open the outlet end of the second flow guide pipe 24. In this case, the gas passes through the housing gas inlet 14 into the head-end chamber 16, passes through the first flow guide pipe 23 into the tail-end chamber 17, and also passes through the second flow guide pipe 24 into the tail-end chamber 17. After the gas passes through the tail-end chamber 17 into the gas pressurization unit gas inlet 41, the gas pressurization unit 40 rotates to accelerate the gas, and the gas flow is then expelled through the gas pressurization unit gas outlet 42.

In another specific embodiment of the present application, referring to Figs. 3 and 4, Fig. 3 shows a schematic structural diagram of the noise reduction box according to the second embodiment of the present application in which the first blocking plate 31 blocks the outlet end of the second flow guide pipe 24, and Fig. 4 shows a schematic structural diagram of the noise reduction box according to the second embodiment of the present application in which the first blocking plate 31 opens the outlet end of the second flow guide pipe 24. The first blocking plate 31 is rotatably connected to the left side of the outlet end of the second flow guide pipe 24. Referring to Fig. 3, when the gas pressurization unit 40 is at a low rotational speed, the gas pressure in the head-end chamber 16 is less than or equal to the reference gas pressure threshold. The first blocking plate 31 blocks the second flow guide pipe 24 under the action of its own weight or, for example, under the action of the elastic force of a torsion spring. Referring to Fig. 3, the gas passes through the housing gas inlet 14 into the head-end chamber 16, passes through the first flow guide pipe 23, an intermediate chamber 18, the first flow guide pipe 23 and the tail-end chamber 17 in sequence, and then enters the gas pressurization unit gas inlet 41. The gas pressurization unit 40 rotates to accelerate the gas, and the gas flow is then expelled through the gas pressurization unit gas outlet 42. Referring to Fig. 4, when the gas pressurization unit 40 is at a relatively high rotational speed, the gas pressure in the head-end chamber 16 is greater than the reference gas pressure threshold, and the force of the gas acting on the first blocking plate 31 can push the first blocking plate 31 to rotate and open the outlet end of the second flow guide pipe 24. The spaced-apart head-end chamber 16 and tail-end chamber 17 are in communication with each other via the second flow guide pipe 24. In this case, the gas passes through the housing gas inlet 14 into the head-end chamber 16, passes through the first flow guide pipe 23, the intermediate chamber 18 and the first flow guide pipe 23 into the tail-end chamber 17, and also passes through the second flow guide pipe 24 into the tail-end chamber 17. After the gas passes through the tail-end chamber 17 into the gas pressurization unit gas inlet 41, the gas pressurization unit 40 rotates to accelerate the gas, and the gas flow is then expelled through the gas pressurization unit gas outlet 42.

In some embodiments of the present application, the actuating assembly includes a second blocking plate 32 and an elastic member 33. The second blocking plate 32 is configured to block the outlet end of the second flow guide pipe 24. One end of the elastic member 33 is connected to the second blocking plate 32, and the other end of the elastic member 33 is connected to the housing 10 or the partition plate 20. When the gas pressure in the reference chamber is greater than the reference gas pressure threshold, the gas pushes the second blocking plate 32 to overcome an elastic force of the elastic member 33 and open the second flow guide pipe 24.

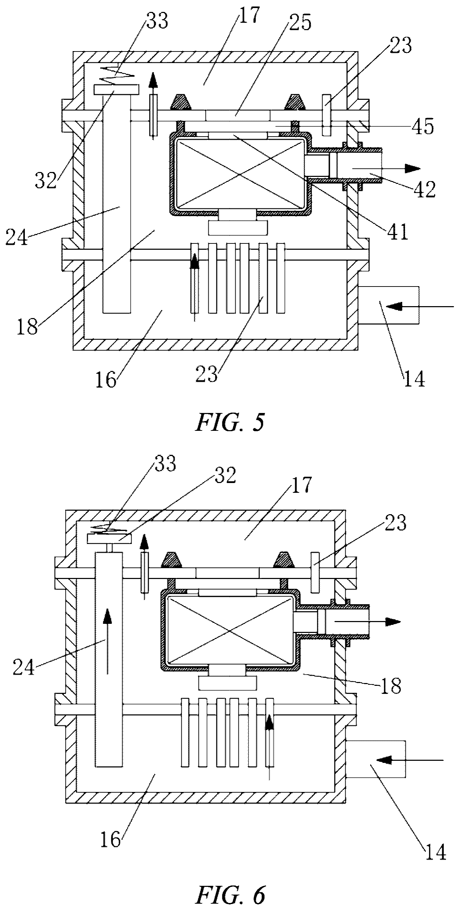

Referring to Figs. 5 and 6, Fig. 5 shows a schematic structural diagram of a noise reduction box according to a third embodiment of the present application in which the second blocking plate 32 blocks the outlet end of the second flow guide pipe 24, and Fig. 6 shows a schematic structural diagram of the noise reduction box according to the third embodiment of the present application in which the second blocking plate 32 opens the outlet end of the second flow guide pipe 24. The head-end chamber 16 is the reference chamber. When the gas pressurization unit 40 is at a low rotational speed, the gas pressure in the head-end chamber 16 is less than or equal to the reference gas pressure threshold. Referring to Fig. 5, the elastic member 33 pushes the second blocking plate 32 to abut against the outlet end of the second flow guide pipe 24, and the second blocking plate 32 blocks the outlet end of the second flow guide pipe 24; and the gas passes through the housing gas inlet 14 into the head-end chamber 16, passes through the first flow guide pipe 23, the intermediate chamber 18, the first flow guide pipe 23 and the tail-end chamber 17 in sequence, and then enters the gas pressurization unit gas inlet 41. The gas pressurization unit 40 rotates to accelerate the gas, and the gas flow is then expelled through the gas pressurization unit gas outlet 42. When the gas pressurization unit 40 is at a relatively high rotational speed, the gas pressure in the head-end chamber 16 is greater than the reference gas pressure threshold. Specifically, referring to Fig. 6, the gas pushes the second blocking plate 32 to overcome the elastic force of the elastic member 33 and open the second flow guide pipe 24. The spaced-apart head-end chamber 16 and tail-end chamber 17 are in communication with each other via the second flow guide pipe 24. In this case, the gas passes through the housing gas inlet 14 into the head-end chamber 16, passes through the first flow guide pipe 23, the intermediate chamber 18 and the first flow guide pipe 23 into the tail-end chamber 17, and also passes through the second flow guide pipe 24 into the tail-end chamber 17. After the gas passes through the tail-end chamber 17 into the gas pressurization unit gas inlet 41, the gas pressurization unit 40 rotates to accelerate the gas, and the gas flow is then expelled through the gas pressurization unit gas outlet 42.

In some embodiments of the present application, the actuating assembly includes a blocking plate, a pressure monitoring element, and an actuating member. The blocking plate is configured to block an inlet end of the second flow guide pipe 24 or an outlet end of the second flow guide pipe 24, and the pressure monitoring element is configured to monitor the gas pressure in the reference chamber and is connected to the actuating member. When the pressure monitoring element detects that the gas pressure in the reference chamber is greater than the reference gas pressure threshold, the actuating member drives the blocking plate to move and open the second flow guide pipe 24.

Specifically, the pressure monitoring element is configured to be disposed in the reference chamber, for example, in the head-end chamber 16, in the tail-end chamber 17, or in the intermediate chamber 18, and directly monitor the gas pressure in the reference chamber. The pressure monitoring element may also be connected to the gas pressurization unit 40 to monitor the rotational speed of the gas pressurization unit 40. When the gas pressurization unit 40 is at a relatively high rotational speed, for example, when the rotational speed of the gas pressurization unit 40 exceeds a reference rotational speed, the pressure monitoring element determines that the gas pressure in the reference chamber is greater than the reference gas pressure threshold. It can be understood that the specific structure of the pressure monitoring element is configured according to usage requirements, and the embodiments of the present application do not limit this.

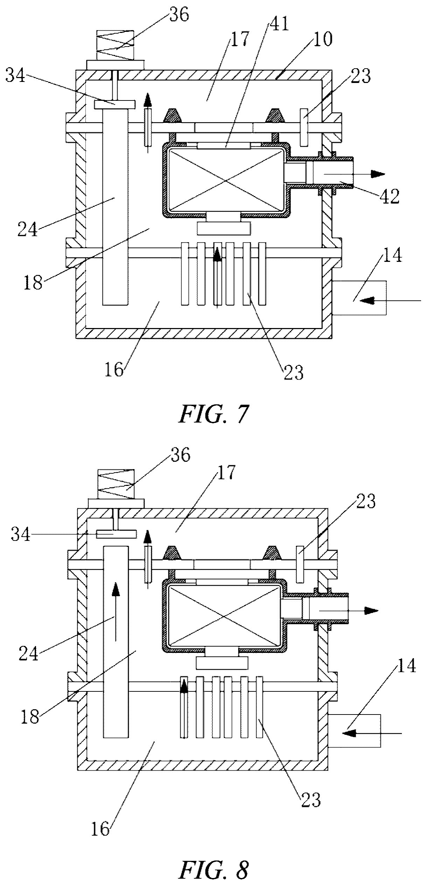

In one specific embodiment of the present application, referring to Figs. 7 and 8, Fig. 7 shows a schematic structural diagram of a noise reduction box according to a fourth embodiment of the present application in which a third blocking plate 34 blocks the outlet end of the second flow guide pipe 24, and Fig. 8 shows a schematic structural diagram of the noise reduction box according to the fourth embodiment of the present application in which the third blocking plate 34 opens the outlet end of the second flow guide pipe 24. The reference chamber is the head-end chamber 16, the tail-end chamber 17, or the intermediate chamber 18. The actuating member includes an actuating motor 36. The actuating motor 36 is connected to the housing 10, a telescopic rod of the actuating motor 36 is connected to the third blocking plate 34, and the third blocking plate 34 is configured to block the inlet end of the second flow guide pipe 24. When the gas pressurization unit 40 is at a low rotational speed, the pressure monitoring element detects that the gas pressure in the reference chamber is less than or equal to the reference gas pressure threshold. Specifically, referring to Fig. 7, the actuating motor 36 does not operate, and the third blocking plate 34 blocks the outlet end of the second flow guide pipe 24; and the gas passes through the housing gas inlet 14 into the head-end chamber 16, passes through the first flow guide pipe 23, the intermediate chamber 18, the first flow guide pipe 23 and the tail-end chamber 17 in sequence, and then enters the gas pressurization unit gas inlet 41. The gas pressurization unit 40 rotates to accelerate the gas, and the gas flow is then expelled through the gas pressurization unit gas outlet 42. When the gas pressurization unit 40 is at a relatively high rotational speed, the pressure monitoring element detects that the gas pressure in the reference chamber is greater than the reference gas pressure threshold. Specifically, referring to Fig. 8, the actuating motor 36 is activated to drive the third blocking plate 34 to move toward the actuating motor 36. The third blocking plate 34 then opens the second flow guide pipe 24. The spaced-apart head-end chamber 16 and tail-end chamber 17 are in communication with each other via the second flow guide pipe 24. In this case, the gas passes through the housing gas inlet 14 into the head-end chamber 16, passes through the first flow guide pipe 23, the intermediate chamber 18 and the first flow guide pipe 23 into the tail-end chamber 17, and also passes through the second flow guide pipe 24 into the tail-end chamber 17. After the gas passes through the tail-end chamber 17 into the gas pressurization unit gas inlet 41, the gas pressurization unit 40 rotates to accelerate the gas, and the gas flow is then expelled through the gas pressurization unit gas outlet 42.

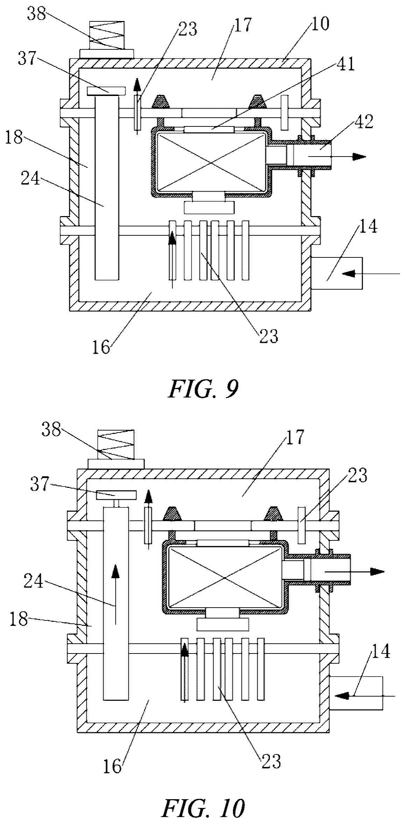

In another specific embodiment of the present application, referring to Figs. 9 and 10, Fig. 9 shows a schematic structural diagram of a noise reduction box according to a fifth embodiment of the present application in which a fourth blocking plate 37 blocks the outlet end of the second flow guide pipe 24, and Fig. 10 shows a schematic structural diagram of the noise reduction box according to the fifth embodiment of the present application in which the fourth blocking plate 37 opens the outlet end of the second flow guide pipe 24. The reference chamber is the head-end chamber 16, the tail-end chamber 17, or the intermediate chamber 18. The actuating member includes an electromagnet 38. The electromagnet 38 is connected to the housing 10. In this case, the fourth blocking plate 37 is a metallic material component. When the gas pressurization unit 40 is at a low rotational speed, the pressure monitoring element detects that the gas pressure in the reference chamber is less than or equal to the reference gas pressure threshold. Specifically, referring to Fig. 9, the electromagnet 38 is de-energized, and the fourth blocking plate 37 blocks the outlet end of the second flow guide pipe 24 under the action of gravity; and the gas passes through the housing gas inlet 14 into the head-end chamber 16, passes through the first flow guide pipe 23, the intermediate chamber 18, the first flow guide pipe 23 and the tail-end chamber 17 in sequence, and then enters the gas pressurization unit gas inlet 41. The gas pressurization unit 40 rotates to accelerate the gas, and the gas flow is then expelled through the gas pressurization unit gas outlet 42. When the gas pressurization unit 40 is at a relatively high rotational speed, the pressure monitoring element detects that the gas pressure in the reference chamber is greater than the reference gas pressure threshold. Referring to Fig. 10, the electromagnet 38 is energized, and generates a magnetic force that attracts the fourth blocking plate 37 to move toward the electromagnet 38. The fourth blocking plate 37 then opens the second flow guide pipe 24. The spaced-apart head-end chamber 16 and tail-end chamber 17 are in communication with each other via the second flow guide pipe 24. In this case, the gas passes through the housing gas inlet 14 into the head-end chamber 16, passes through the first flow guide pipe 23, the intermediate chamber 18 and the first flow guide pipe 23 into the tail-end chamber 17, and also passes through the second flow guide pipe 24 into the tail-end chamber 17. After the gas passes through the tail-end chamber 17 into the gas pressurization unit gas inlet 41, the gas pressurization unit 40 rotates to accelerate the gas, and the gas flow is then expelled through the gas pressurization unit gas outlet 42.

In some embodiments of the present application, referring to Fig. 3, the gas pressurization unit 40 is disposed in the intermediate chamber 18. A gas pressurization unit motor 46 is provided corresponding to the first flow guide pipe 23. When the gas passes through the head-end chamber 16 and the first flow guide pipe 23 into the intermediate chamber 18, the gas can cool the gas pressurization unit motor 46, to maintain the gas pressurization unit 40 at a relatively desirable ambient temperature. The gas then flows from the first flow guide pipe 23 into the tail-end chamber 17.

In some embodiments of the present application, the blocking plate is an elastic material component. That is, the first blocking plate 31, the second blocking plate 32, the third blocking plate 34 and the fourth blocking plate 37 described above are elastic material components. The elastic material component may be, for example, a silicone rubber valve plate, a metal elastic piece, or a silicone rubber elastic piece, which may be specifically selected according to usage requirements, and the embodiments of the present application do not limit this.

In some embodiments of the present application, the flow guide pipes 26 extend out from a surface of the partition plate 20, i.e., both the first flow guide pipe 23 and the second flow guide pipe 24 extend out from the surface of the partition plate 20. When the gas enters the gas pressurization unit 40, noise is generated at the gas pressurization unit gas inlet 41. During the outward propagation, the noise passes through the chambers 15 and the flow guide pipes 26. Portions of the flow guide pipes 26 that extend out from the partition plate 20 can block the noise, to convert part of acoustic energy of the noise into thermal energy, thereby weakening the acoustic energy and thus reducing the noise.

In some embodiments of the present application, any one of partition plates 20 is provided with at least two first flow guide pipes 23 to obtain a corresponding ventilation area, thereby achieving the purpose of noise reduction.

In some embodiments of the present application, the smaller the inner diameter of the first flow guide pipe 23, the better the noise reduction effect. For example, the inner diameter of the first flow guide pipe 23 ranges from 1 mm to 10 mm, preferably from 4 mm to 6 mm. Specifically, for example, the inner diameter of the first flow guide pipe 23 is 3 mm, 5 mm, 7 mm, 8 mm, 9 mm, 5.5 mm, etc.

In some embodiments of the present application, the number of second flow guide pipes 24 is less than the number of first flow guide pipes 23.

In some embodiments of the present application, the inner diameter of the first flow guide pipe 23 is smaller than that of the second flow guide pipe 24. When the second flow guide pipe 24 is open, the ventilation area in the noise reduction box can be effectively increased, thereby reducing the overall gas resistance, lowering the gas flow velocity, and reducing the secondary eddy noise and high-frequency noise caused by the high gas flow velocity, so as to achieve the purpose of noise reduction.

In some embodiments of the present application, the housing 10 is a plastic material component, which can reduce the noise caused by the vibration of the gas pressurization unit 40.

In some embodiments of the present application, at least two partition plates 20 are disposed in parallel and spaced apart.

Referring further to Figs. 3 and 4, in an example where two partition plates 20 are provided, the two partition plates 20 include a first partition plate 21 and a second partition plate 22. The first partition plate 21 is arranged between an upper housing 11 and an intermediate housing 12, and the second partition plate 22 is arranged between the intermediate housing 12 and a lower housing 13. The first partition plate 21 is provided with at least two first flow guide pipes 23, and the second partition plate 22 is provided with at least six first flow guide pipes 23. The second flow guide pipe 24 is connected to the first partition plate 21 and the second partition plate 22. When the gas pressurization unit 40 is at a low rotational speed, the gas pressure in the head-end chamber 16 is less than or equal to the reference gas pressure threshold. As shown in Fig. 3, the first blocking plate 31 blocks the second flow guide pipes 24; and the gas passes through the housing gas inlet 14 into the head-end chamber 16, passes through the first flow guide pipe 23, the intermediate chamber 18, the first flow guide pipe 23 and the tail-end chamber 17 in sequence, and then enters the gas pressurization unit gas inlet 41. When the gas pressurization unit 40 is at a relatively high rotational speed, the gas pressure in the head-end chamber 16 is greater than the reference gas pressure threshold. As shown in Fig. 4, the force of the gas acting on the first blocking plate 31 can push the first blocking plate 31 to rotate and open the outlet end of the second flow guide pipe 24, and thus the head-end chamber 16 is in communication with the tail-end chamber 17. In this case, the gas passes through the housing gas inlet 14 into the head-end chamber 16, passes through the first flow guide pipe 23, the intermediate chamber 18 and the first flow guide pipe 23 into the tail-end chamber 17, and also passes through the second flow guide pipe 24 into the tail-end chamber 17. After the gas passes through the tail-end chamber 17 into the gas pressurization unit gas inlet 41, the gas pressurization unit 40 rotates to accelerate the gas, and the gas flow is then expelled through the gas pressurization unit gas outlet 42.

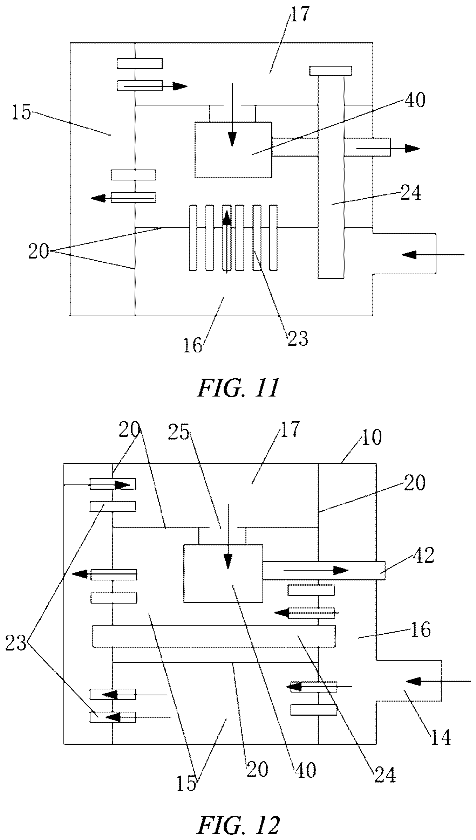

In some embodiments of the present application, referring to Fig. 11, two partition plates 20 are disposed horizontally and spaced apart, and a third partition plate 20 is disposed vertically and is connected to the two partition plates 20. The gas flow direction is shown by the arrows in Fig. 11.

In some embodiments of the present application, referring to Fig. 12, two partition plates 20 are disposed horizontally and spaced apart, and two further partition plates 20 are disposed vertically and spaced apart and are connected to the two partition plates 20 disposed horizontally. The gas flow direction is shown by the arrows in Fig. 12.

It can be understood that the specific installation positions and quantity of the partition plates 20 can be configured according to usage requirements, and the embodiments of the present application do not limit this.

The noise reduction box in the embodiments of the present application does not include sound-absorbing cotton, thereby avoiding potential risks caused by the decomposition of fine particles from the sound-absorbing cotton and providing improved safety. The noise reduction box can change the gas path and adjust the ventilation area according to the change in the rotational speed of the gas pressurization unit 40, thus achieving a better silencing effect. Moreover, while achieving noise reduction, the noise reduction box reduces the output power of the gas pressurization unit 40, alleviates the load on the gas pressurization unit 40, improves the efficiency of the gas pressurization unit 40, lowers the temperature of the gas pressurization unit 40, achieves more stable operation of the gas pressurization unit 40, and prolongs the service life of the gas pressurization unit 40.

The embodiments of the present application further provide a ventilation therapy apparatus. The ventilation therapy apparatus includes a gas pressurization unit 40 and the noise reduction box described above. The gas pressurization unit 40 is disposed in the noise reduction box.

When the gas pressurization unit 40 rotates, noise is generated at the gas pressurization unit gas inlet 41. During outward propagation, the noise is blocked by the partition plate 20, the chambers 15, and the flow guide pipes 26, thereby effectively reducing the noise. Moreover, the noise reduction box can adjust the ventilation area according to the rotational speed of the gas pressurization unit 40, achieving a better silencing effect. While achieving noise reduction, the noise reduction box reduces the output power of the gas pressurization unit 40, alleviates the load on the gas pressurization unit 40, improves the efficiency of the gas pressurization unit 40, lowers the temperature of the gas pressurization unit 40, achieves more stable operation of the gas pressurization unit 40, and prolongs the service life of the gas pressurization unit 40, thereby effectively improving user satisfaction.

In some embodiments of the present application, the ventilation therapy apparatus further includes a shock-absorbing sleeve 43. An outer side of the gas pressurization unit 40 is enclosed by the shock-absorbing sleeve 43. A suspension end 44 is provided at an upper end of the shock-absorbing sleeve 43. The gas pressurization unit 40 is suspended from the partition plate 20 via the suspension end 44. A portion of the suspension end 44 located between the partition plate 20 and the gas pressurization unit 40 defines an air intake channel 45. The air intake channel 45 has one end connected to a partition plate through hole 25 in the partition plate 20, and the other end connected to the gas pressurization unit gas inlet 41. The provision of the shock-absorbing sleeve 43 can ensure that the gas pressurization unit 40 is in the correct position while providing shock absorption and noise reduction effects.

In some embodiments of the present application, the shock-absorbing sleeve 43 is a silicone material component, a soft rubber material component, etc., and the embodiments of the present application are not limited to this.

It should be noted that, for brief description, the method embodiments are represented as a series of actions. However, those skilled in the art should appreciate that the embodiments of the present application are not limited to the described order of the actions, because according to the embodiments of the present application, some steps may be performed in another order or simultaneously. It should be further appreciated by those skilled in the art that the embodiments described in the specification all belong to preferred embodiments, and the involved actions are not necessarily required by the embodiments of the present application.

It should be noted that the terms "comprise", "include", or any other variant thereof herein is intended to encompass a non-exclusive inclusion, such that a process, method, article, or apparatus that includes a series of elements not only comprises those elements, but also comprises other elements not explicitly listed, or elements that are inherent to such a process, method, article, or apparatus. In the absence of more restrictions, the element defined by the phrase "comprising a/an ..." does not exclude the presence of a further identical element in the process, method, article, or apparatus that includes the element.

The embodiments of the present application are described above with reference to the drawings. However, the present application is not limited to the aforementioned particular embodiments. The aforementioned particular embodiments are merely for illustration rather than limitation. In light of the teachings of the present application, a person of ordinary skill in the art may further use various forms without departing from the spirit and the protection scope of the claims of the present application, and these forms all fall within the protection scope of the present application.

## Claims

1. A noise reduction box, comprising a housing (10), a first flow guide pipe (23), and a second flow guide pipe (24), wherein at least two chambers (15) are provided in the housing (10);
the first flow guide pipe (23) and the second flow guide pipe (24) are both configured to guide the flow of a gas, so that the gas flows from one of the chambers (15) to another one of the chambers (15); wherein
when a gas pressure in a reference chamber is less than or equal to a reference gas pressure threshold, the first flow guide pipe (23) guides the flow of the gas; and
when the gas pressure in the reference chamber is greater than the reference gas pressure threshold, the first flow guide pipe (23) and the second flow guide pipe (24) jointly guide the flow of the gas.

2. The noise reduction box according to claim 1, further comprising an actuating assembly connected to the second flow guide pipe (24), wherein the actuating assembly is configured to block the second flow guide pipe (24) when the gas pressure in the reference chamber is less than or equal to the reference gas pressure threshold, and to open the second flow guide pipe (24) when the gas pressure in the reference chamber is greater than the reference gas pressure threshold.

3. The noise reduction box according to claim 2, wherein an outlet end of the second flow guide pipe (24) is oriented upward; the actuating assembly comprises a blocking plate rotatably connected to the outlet end of the second flow guide pipe (24); and
when the gas pressure in the reference chamber is greater than the reference gas pressure threshold, the gas pushes the blocking plate to rotate and open the second flow guide pipe (24).

4. The noise reduction box according to claim 2, wherein the actuating assembly comprises a blocking plate and an elastic member (33); the blocking plate is configured to block an outlet end of the second flow guide pipe (24); one end of the elastic member (33) is connected to the blocking plate, and the other end of the elastic member (33) is connected to the housing (10); and when the gas pressure in the reference chamber is greater than the reference gas pressure threshold, the gas pushes the blocking plate to overcome an elastic force of the elastic member (33) and open the second flow guide pipe (24).

5. The noise reduction box according to claim 2, wherein the actuating assembly comprises a blocking plate, a pressure monitoring element and an actuating member; the blocking plate is configured to block an inlet end of the second flow guide pipe (24) or an outlet end of the second flow guide pipe (24); the pressure monitoring element is configured to monitor the gas pressure in the reference chamber and is connected to the actuating member; and
when the pressure monitoring element detects that the gas pressure in the reference chamber is greater than the reference gas pressure threshold, the actuating member drives the blocking plate to move and open the second flow guide pipe (24).

6. The noise reduction box according to any one of claims 3 to 5, wherein the blocking plate is an elastic material component.

7. The noise reduction box according to claim 1, further comprising a partition plate (20), wherein the partition plate (20) is disposed in the housing (10), and is connected to the housing (10), the first flow guide pipe (23) and the second flow guide pipe (24); the partition plate (20) divides the housing (10) into the at least two chambers (15); and
the first flow guide pipe (23) and the second flow guide pipe (24) both extend out from a surface of the partition plate (20).

8. The noise reduction box according to claim 7, wherein the first flow guide pipe (23) is connected to one of partition plates (20), and the second flow guide pipe (24) is connected to at least one of the partition plates (20).

9. The noise reduction box according to claim 7, wherein an inner diameter of the first flow guide pipe (23) is smaller than an inner diameter of the second flow guide pipe (24).

10. The noise reduction box according to claim 7, wherein any one of partition plates (20) is provided with at least two first flow guide pipes (23).

11. The noise reduction box according to claim 7, wherein at least two partition plates (20) are arranged in parallel and spaced apart.

12. The noise reduction box according to claim 1, wherein the housing (10) is a plastic material component.

13. A ventilation therapy apparatus, comprising a gas pressurization unit (40) and a noise reduction box according to any one of claims 1 to 12, wherein the gas pressurization unit (40) is disposed in the noise reduction box.

14. The ventilation therapy apparatus according to claim 13, further comprising a shock-absorbing sleeve (43), wherein an outer side of the gas pressurization unit (40) is enclosed by the shock-absorbing sleeve (43), and a suspension end (44) is provided at an upper end of the shock-absorbing sleeve (43); the gas pressurization unit (40) is suspended from the partition plate (20) via the suspension end (44), and a portion of the suspension end (44) located between the partition plate (20) and the gas pressurization unit (40) defines an air intake channel (45); and the air intake channel (45) has one end connected to a partition plate through hole (25) in the partition plate (20), and the other end connected to a gas pressurization unit gas inlet (41).
